(19) Europäisches Patentamt
European Patent Office
Office européen des brevets

(11) Veröffentlichungsnummer: **0 224 442 B1**

(12) # EUROPÄISCHE PATENTSCHRIFT

(45) Veröffentlichungstag der Patentschrift: **16.05.90**

(51) Int. Cl.[5]: **C08K 5/37**, C07C 323/00, C10M 135/24

(21) Anmeldenummer: **86810511.5**

(22) Anmeldetag: **07.11.86**

(54) **Substituierte Phenole als Stabilisatoren.**

(30) Priorität: **13.11.85 CH 4871/85**
**24.09.86 CH 3828/86**

(43) Veröffentlichungstag der Anmeldung:
**03.06.87 Patentblatt 87/23**

(45) Bekanntmachung des Hinweises auf die Patenterteilung:
**16.05.90 Patentblatt 90/20**

(84) Benannte Vertragsstaaten:
**BE CH DE ES FR GB IT LI NL**

(56) Entgegenhaltungen:
**EP-A- 0 165 209**
**GB-A- 1 184 533**
**US-A- 3 903 173**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel(CH)**

(72) Erfinder: **Meier, Hans-Rudolf, Dr., Rte du Confin 54, CH-1723 Marly(CH)**
Erfinder: **Knobloch, Gerrit, Dr., Ringstrasse 33, CH-4422 Arisdorf(CH)**

**EP 0 224 442 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft Zusammensetzungen enhaltend ein substituiertes Bis-(alkylthiomethyl)-phenol als Stabilisator sowie neue Bis-(alkylthiomethyl)-phenole.

Alkylthiomethylhaltige Phenole sind bekannt als Stabilisatoren. So ist beispielsweise in der US-PS 3 660 352 der Einsatz von 2,4,6-Trialkyl-bis-(3,5-alkylthiomethyl)-phenolen als Antioxidantien in Polymeren und Elastomeren beschrieben. Aus der US-PS 3 227 677 sind ferner 2,6-Bis-(alkoxycarbonylalkylenthiomethyl)-4-alkylphenole als Antioxidantien für Polyolefine bekannt.

Nach der GB-PS 1 184 533 sind 2,4-Bis-(alkylthiomethyl)-3,6-dialkylphenole als Stabilisatoren für organische Polymere, wie z.B. Styrol/Butadien-Copolymere, ABS-Harze, MBS-Harze, und SAN-Harze, sowie für synthetische Öle einsetzbar.

Es besteht weiterhin ein Bedarf an wirksamen Stabilisatoren für Materialien, welche gegen thermischen, oxidativen oder lichtinduzierten Abbau empfindlich sind.

Gegenstand der Erfindung ist daher eine Zusammensetzung ethaltend ein Elastomer oder ein klebrigmachendes Harz und mindestens eine Verbindung der Formel I

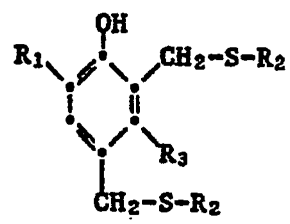

(I),

worin

$R_1$ $C_1$-$C_8$-Alkyl oder $C_5$-$C_{12}$-Cycloalkyl bedeutet,

die Reste $R_2$ unabhängig voneinander für $C_1$-$C_{18}$-Alkylgruppen stehen, welche unsubstituiert sind oder durch 1 oder 2 Hydroxylgruppen oder durch $-OCO-R_4$, $-CO-OR_5$ oder $-CO-NR_5R_6$ substituiert sind, oder Phenyl oder Benzyl bedeuten,

$R_3$ Wasserstoff oder Methyl bedeutet, unter der Bedingung, dass, wenn $R_1$ von Methyl verschieden ist, $R_3$ für Methyl steht,

$R_4$ $C_1$-$C_{17}$-Alkyl, $C_2$-$C_8$-Alkenyl oder ein Rest der Formel Ia ist

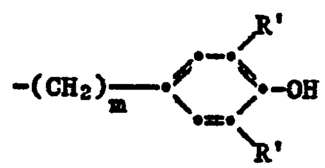

(Ia),

m für 0, 1 oder 2 steht, und die Reste

R' unabhängig voneinander die für $R_1$ genannte Bedeutung haben,

$R_5$ ein gegebenenfalls durch -O-, -S- oder -NR'- unterbrochenes $C_1$-$C_{20}$-Alkyl ist, und

$R_6$ für Wasserstoff steht oder eine für $R_5$ genannte Bedeutung besitzt.

$R_1$ und R' als $C_1$-$C_8$-Alkyl bedeuten beispielsweise Methyl, Aethyl, n-Propyl, Isopropyl, n-Butyl, sek.-Butyl, t-Butyl, n-Pentyl, Isopentyl, n-Hexyl, 1,1-Dimethylbutyl, n-Heptyl, n-Octyl oder 2-Aethylhexyl.

Bevorzugt sind $R_1$ und R' $C_1$-$C_4$-Alkyl, insbesondere Methyl, Aethyl, Isopropyl oder t-Butyl, und ganz besonders Methyl oder t-Butyl.

$R_1$ und R' als $C_5$-$C_{12}$-Cycloalkyl bedeuten beispielsweise Cyclopentyl, Cyclohexyl, Cycloheptyl, Cyclooctyl oder Cyclododecyl. Bevorzugt sind $R_1$ und R' $C_5$-$C_8$-Cycloalkyl, insbesondere Cyclohexyl.

$R_2$ als $C_1$-$C_{18}$-Alkyl bedeutet beispielsweise zusätzlich zu den für $R_1$ erwähnten Resten noch n-Nonyl, n-Decyl, 1,1,3,3-Tetramethylbutyl, 1,1,3,3-Tetramethylhexyl, n-Undecyl, n-Dodecyl, 1,1,3,3,5,5-Hexamethylhexyl, n-Tetradecyl, n-Hexadecyl oder n-Octadecyl. Bevorzugt ist $R_2$ $C_4$-$C_{18}$-, insbesondere $C_8$-$C_{18}$-Alkyl, z.B. n-Octyl oder n-Dodecyl.

$R_2$ als $C_1$-$C_{18}$-Alkyl kann durch eine oder zwei Hydroxylgruppen substituiert sein. Als solche Reste kommen beispielsweise 2-Hydroxyäthyl, 2-Hydroxypropyl, 2-Hydroxybutyl, 2-Hydroxydodecyl, 2-Hydroxyhexadecyl, 2-Hydroxyoctadecyl oder 2,3-Dihydroxypropyl in Frage. Bevorzugt ist 2-Hydroxyäthyl.

$R_4$ als $C_1$-$C_{17}$-Alkyl bedeutet beispielsweise die für $R_2$ genannten Reste sowie Methyl, vorzugsweise $C_1$-$C_{12}$-Alkyl, wie zum Beispiel Methyl, Aethyl, n-Propyl, Isopropyl, n-Butyl, t-Butyl, n-Hexyl, n-Octyl, 2-Aethylhexyl oder n-Dodecyl.

Bedeutet $R_4$ $C_2$-$C_8$-Alkenyl, so kommen beispielsweise die Reste Vinyl, Allyl, Methallyl, But-3-enyl, Pent-4-enyl, Hex-5-enyl, Oct-7-enyl, Dec-9-enyl oder Dodec-11-enyl in Frage. Bevorzugt ist $R_4$ Vinyl oder Allyl.

$R_4$ als ein Rest der Formel Ia kann beispielsweise 4-Hydroxy-3,5-dimethylphenyl, 4-Hydroxy-3,5-di-t-butylphenyl, 4-Hydroxy-3,5-dimethylbenzyl, 4-Hydroxy-3,5-di-t-butylbenzyl oder 4-Hydroxy-3,5-dimethylphenyläthyl, bevorzugt 4-Hydroxy-3,5-di-t-butylbenzyl, bedeuten.

$R_5$ oder $R_6$ als $C_1$-$C_{20}$-Alkyl bedeutet beispielsweise die für $R_2$ genannten Reste sowie Methyl, Octadecyl oder Eicosyl, vorzugsweise Methyl, Aethyl, n-Hexyl, n-Octyl oder n-Dodecyl.

$R_5$ oder $R_6$ als durch -O-, -S- oder -NR'- unterbrochenes $C_1$-$C_{20}$-Alkyl bedeutet beispielsweise 3-Oxabutyl, 3-Oxapentyl, 3,6-Dioxaheptyl, 3,6,9-Trioxadecyl, 3,6,9,12-Tetraoxatridecyl, 3,6,9,12,15-Pentaoxahexadecyl, 3,6,9,12,15,18-Hexaoxanonadecyl, 3-Thiabutyl, 3-Azabutyl, 3-Methyl-3-azapentyl, 3-Aethyl-3-azapentyl, 3-Methyl-3-aza-6-oxaheptyl, vorzugsweise 3-Oxapentyl oder 3-Azabutyl.

Bevorzugt werden Zusammensetzungen, welche ein Elastomer und mindestens eine Verbindung der Formel I enthalten.

Besonders bevorzugt werden Zusammensetzungen enthaltend ein Elastomer und mindestens eine Verbindung der Formel I, worin die Reste $R_2$ unabhängig voneinander für $C_2$-$C_{18}$-Alkylgruppen stehen, welche unsubstituiert sind oder durch 1 oder 2 Hydroxylgruppen oder durch -OCO-$R_4$, -CO-O$R_5$ oder -CO-N$R_5R_6$ substituiert sind, wobei $R_4$, $R_5$ und $R_6$ die zuvor genannte Bedeutung haben.

Ganz besonders bevorzugt werden Zusammensetzungen enthaltend ein Elastomer oder ein klebrigmachendes Harz, worin $R_1$ in der Formel I $C_1$-$C_4$-Alkyl ist, insbesondere Methyl oder t-Butyl.

Ebenfalls bevorzugt werden Zusammensetzungen enthaltend ein Elastomer oder ein klebrigmachendes Harz, worin $R_2$ in der Formel I $C_4$-$C_{18}$-Alkyl, $-CH_2CH_2-OH$, $-CH_2CH_2-O-CO-R_4$, $-CH_2-CO-R_5$ oder $-CH_2CH_2-CO-R_5$ bedeutet, wobei $R_4$ und $R_5$ die zuvor genannte Bedeutung haben. Besonders bevorzugt ist $R_2$ $C_4$-$C_{18}$-Alkyl oder $-CH_2-COO-R_6$, wobei $R_6$ die zuvor genannte Bedeutung hat. Ganz besonders bevorzugt ist $R_2$ $C_4$-$C_{18}$-Alkyl.

Als Beispiele für Vertreter von Verbindungen der Formel I sind die nachfolgend aufgezählten Substanzen anzusehen:

2,4-Bis(2'-hydroxyäthylthiomethyl)-6-methyl-phenol,
2,4-Bis(2',3'-dihydroxypropylthiomethyl)-3,6-dimethyl-phenol,
2,4-Bis(2'-acetyloxyäthylthiomethyl)-3,6-dimethyl-phenol,
2,4-Bis(2'-n-decanoyloxyäthylthiomethyl)-6-methylphenol,
2,4-Bis(n-octylthiomethyl)-6-methyl-phenol,
2,4-Bis(t-octylthiomethyl)-6-methyl-phenol,[1]
2,4-Bis(t-dodecylthiomethyl)-6-methyl-phenol,[2]
2,4-Bis(benzylthiomethyl)-6-methyl-phenol,
2,4-Bis(phenylthiomethyl)-3-methyl-6-t-butyl-phenol,
2,4-Bis(2'-äthylhexyloxycarbonylmethyl-thiomethyl)-6-methyl-phenol,
2,4-Bis(n-octadecyloxycarbonylmethyl-thiomethyl)-3,6-dimethylphenol,
2,4-Bis[2'-(2''-äthylhexyloxycarbonyl)-äthylthiomethyl]-3-methyl-6-t-butyl-phenol.

---

[1] t-Octyl ist 1,1,3,3-Tetramethylbutyl
[2] t-Dodecyl ist ein Gemisch aus 1,1,3,3,5,5-Hexamethylhexyl und 1,1,4,6,6-Pentamethylhept-(4)-yl

Als Elastomeres können die erfindungsgemässen Zusammensetzungen beispielsweise folgende Materialien enthalten:

1. Polydiene, wie beispielsweise Polybutadien, Polyisopren oder Polychloropren; Blockpolymere, wie beispielsweise Styrol/Butadien/Styrol, Styrol/Isopren/Styrol oder Acrylnitril/Butadien-Copolymere.
2. Copolymere von Mono- und Diolefinen untereinander oder mit anderen Vinylmonomeren, wie z.B. Aethylen-Alkylacrylat-Copolymere, Aethylen-Alkylmethacrylat-Copolymere, Aethylen-Vinylacetat-Copolymere sowie Terpolymere von Aethylen mit Propylen und einem Dien, wie Hexadien, Dicyclopentadien oder Aethylidennorbornen.
3. Halogenhaltige Polymere, wie z.B. Polychloropren, Chlorkautschuk, chloriertes oder chlorsulfoniertes Polyäthylen, Epichlorhydrin-Homo- und -Copolymere, Chlortrifluoroäthylen-Copolymere, Polymere aus halogenhaltigen Vinylverbindungen, wie z.B. Polyvinylidenchlorid, Polyvinylidenfluorid; sowie deren Copolymere, wie Vinylchlorid-Vinylidenchlorid, Vinylchlorid-Vinylacetat oder Vinylidenchlorid-Vinylacetat.
4. Polyurethane, die sich von Polyäthern, Polyestern und Polybutadien mit endständigen Hydroxylgruppen einerseits und aliphatischen oder aromatischen Polyisocyanaten andererseits ableiten sowie deren Vorprodukte.
5. Naturkautschuk.
6. Mischungen (Polyblends) der vorgenannten Polymeren.
7. Wässrige Emulsionen natürlicher oder synthetischer Kautschuke, wie z.B. Naturkautschuk-Latex oder Latices von carboxylierten Styrol-Butadien-Copolymeren.

Gegebenenfalls liegen diese Elastomere als Latices vor und können als solche stabilisiert werden.

Bevorzugt sind Zusammensetzungen, welche als Elastomeres ein Polydien, wie Polybutadien-Kautschuk, ein halogenhaltiges Polymer, wie Polyvinylidenfluorid, oder ein Polyurethan enthalten.

Die erfindungsgemässen Zusammensetzungen enthalten zweckmässig 0,01-10 Gew.-% des Stabilisators der Formel I, bezogen auf das Elastomer, insbesondere 0,05-5,0 Gew.-%. Gemische von Stabilisatoren der Formel I können ebenfalls eingesetzt werden.

Die Einarbeitung in die Elastomeren kann beispielsweise durch Einmischen der Substanzen der Formel I und gegebenenfalls weiterer Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels erfolgen. Die Verbindungen der Formel I können auch in Form eines Masterbatches, der diese Verbindungen beispielsweise in einer Konzentration von 2,5 bis 25 Gew.-% enthält, den zu stabilisierenden Kunststoffen zugesetzt werden.

Als klebrigmachende Harze können die erfindungsmässigen Zusammensetzungen beispielsweise folgende Materialien enthalten:
- Natürliche Kolophoniumharze (rosins) wie gum rosin, wood rosin, tall oil rosin.
- Derivate der Kolophoniumharze, wie Glycerolester, Pentaerythritester, jeweils hydriert oder nicht hydriert, disproportioniert oder nicht disproportioniert
- Synthetische $C_5$ - oder $C_9$-Kohlenwasserstoffharze
- Indenharze, Methylindenharze, Cumaron-Indenharze
- Terpenharze
- Methylstyrolharze
- Phenolharze
- weitere Klebrigmacher wie Asphalt oder Bitumen
- Mischungen vorgenannter Harze
- wässrige Emulsionen vorgenannter Harze.

Bevorzugt sind Zusammensetzungen, welche ein synthetisches $C_5$- oder $C_9$-Kohlenwasserstoffharz, ein Kolophoniumharz oder ein Derivat von Kolophoniumharzen enthalten.

Die klebrigmachenden Harzzusammensetzungen enthalten zweckmässig 0,01-10 Gew.-% des Stabilisators der Formel I, bezogen auf das klebrigmachende Harz, insbesondere 0,03-3,0 Gew.-%. Gemische von Stabilisatoren der Formel I können ebenfalls eingesetzt werden.

Die Einarbeitung der Verbindungen der Formel I in die klebrigmachenden Harze kann nach den in der Technik üblichen Methoden, vor, während oder nach der Herstellung oder Modifizierung erfolgen.

Die erfindungsgemässen Zusammensetzungen können in verschiedenster Form angewendet werden, z.B. als Folien, Fasern, Bändchen, Formmassen, Profile oder als Bindemittel für Lacke, Klebstoffe oder Kitte.

Die Verbindungen der Formel I sind teilweise bekannt.

Die Verbindungen der Formel I, welche neu sind, sind ebenfalls Gegenstand der vorliegenden Erfindung und entsprechen der Formel II.

Die Erfindung betrifft daher auch Verbindungen der Formel II

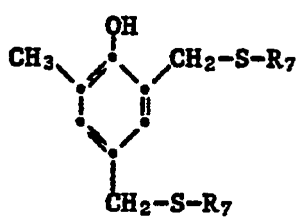

(II),

worin die Reste $R_7$ unabhängig voneinander für $C_8$-$C_{18}$-Alkylgruppen stehen, welche unsubstituiert sind oder durch 1 oder 2 Hydroxylgruppen oder durch $-OCO-R_4$, $-CO-OR_5$ oder $-CO-NR_5R_6$ substituiert sind, wobei $R_4$, $R_5$ und $R_6$ die bereits genannte Bedeutung haben.

Die bereits zuvor gegebene beispielhaften und bevorzugten Bedeutungsmöglichkeiten für $R_2$ in der Formel I gelten sinngemäss auch für $R_7$ in der Formel II. Das gleiche gilt für die Symbole $R_4$, $R_5$ und $R_6$.

Bevorzugt sind Verbindungen der Formel II, worin die Reste $R_7$ unabhängig voneinander für unsubstituiertes $C_8$-$C_{18}$-Alkyl stehen.

Besonders bevorzugt sind Verbindungen der Formel II, worin die Reste $R_7$ die gleiche Bedeutung haben und für $C_8$-$C_{12}$-Alkyl stehen.

Die Herstellung der bekannten und neuen Verbindungen der Formeln I und II erfolgt nach an sich bekannten Methoden, wie sie beispielsweise in der GB-A-1 184 533 und in der US-A-3 227 677 beschrieben sind. Alle Ausgangsprodukte sind bekannte Verbindungen und können nach bekannten Verfahren hergestellt werden. Sie sind zum Teil auch im Handel erhältlich.

Ein weiterer Gegenstand der vorliegenden Erfindung ist ein Verfahren zur Herstellung von Verbindungen der Formel II, das dadurch gekennzeichnet ist, dass man 2,4-Bis(dimethylaminomethyl)-6-methylphenol mit einem Merkaptan $R_7$-SH umsetzt.

Die erfindungsgemässen Phenole der Formel II eignen sich - neben dem Stabilisieren von Elastomeren und klebrigmachenden Harze - auch zum Stabilisieren von Schmierstoffen auf der Basis von Mineralölen oder synthetischen Oelen, insbesondere von Motorenölen.

Die in Frage kommenden Schmierstoffe sind dem Fachmann geläufig und z.B. im "Schmiermittel Taschenbuch (Hüthig Verlag, Heidelberg, 1974)" beschrieben.

Ein weiterer Gegenstand der Erfindung ist daher die Verwendung von Verbindungen der Formel II als Stabilisatoren für Schmierstoffe gegen dessen Schädigung durch Einwirkung von Sauerstoff, Wärme, Licht und energiereiche Strahlung.

Die Erfindung betrifft ferner Zusammensetzungen enthaltend einen Schmierstoff und mindestens eine Verbindung der Formel II.

Die erfindungsgemässen Phenole der Formel II werden zweckmässigerweise in einer Konzentration von 0,01-10 Gew.-%, berechnet auf den Schmierstoff, zugesetzt. Vorzugsweise werden 0,05 bis 5,0 Gew.-%, besonders bevorzugt 0,1 bis 2,0 Gew.-% der Phenole, berechnet auf den Schmierstoff, verwendet.

Gemische von Phenolen der Formel II können ebenfalls eingesetzt werden.

Erfindungsgemässe Schmierstoff-Formulierungen enthaltend Phenole der Formel II können zusätzlich noch andere Additive enthalten, die zugegeben werden, um gewisse Gebrauchseigenschaften zu verbessern, wie z.B. weitere Antioxidantien, Metallpassivatoren, Rostinhibitoren, Viskositäts-Index-Verbesserer, Stockpunkterniedriger, Dispergiermittel/Tenside und Verschleissschutzadditive.

Als weitere Additive, mit denen zusammen die erfindungsgemässen Phenole der Formel II in Schmierstoffen eingesetzt werden können, sind beispielsweise zu nennen:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tricyclohexylphenol, 2,6-Di-tert.butyl-4-methoxymethylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2'-Thio-bis-(6-tert.butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenyl], 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Di-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat], Di-(3-tert.butyl-4-hydroxy-5-methylphenyl)-dicyclopentadien, Di-[2-(3'-tert.butyl-2'-hydroxy-5'-methyl-benzyl)-6-tert.butyl-4-methylphenyl]-terephthalat.

1.5 Benzylverbindungen, z.B. 1,3,5-Tri-(3,5-di-tert.butyl-4-hydroxybenzyl)-2,4,6-trimethylbenzol, Di-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)-dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, 3,5-Di-tert.butyl-4-hydroxybenzyl-phosphonsäure-monoethylester, Calcium-salz.

1.6. Acylaminophenole, z.B. 4-Hydroxy-laurinsäureanilid, 4-Hydroxystearinsäureanilid, 2,4-Bis-octylmercapto-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat, Di-hydroxyethyl-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat, Di-hydroxyethyl-oxyalsäurediamid.

1.9. Amide der β-(3,5-Di-tert.butyl-4-hydroxyphenyl)-propionsäure, wie z.B. N,N'-Di-(3,5-di-tert.butyl-4-hydroxyphenylpropionyl)-hexamethylendiamin, N,N'-Di-(3,5-di-tert.butyl-4-hydroxyphenylpropionyl)-trimethylendiamin, N,N'-Di-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3', 5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenylundecylketonoxim, Nickelkomplexe des 1-Phenyl-4-lauroyl-5-hydroxypyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-di-methylamino-propyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o-und p-Methoxy- sowie von o- und p-Ethoxy-di-substituierten Oxaniliden.

3. Metalldesaktivatoren, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

In der Praxis können auch die erfindungsgemässen Zusammensetzungen enthaltend ein Elastomer oder ein klebrigmachendes Harz und Phenole der Formel I weitere Additive enthalten. Beispiele für solche Additive sind:

1. Antioxidantien

Beispiele für Antioxidantien sind unter den obigen Punkten 1 bis 10 betreffend zusätzliche Additive für Schmierstoffe zu entnehmen.

2. UV-Absorber und Lichtschutzmittel

2.1. 2-(2'-Hydroxyphenyl)-benztriazole, wie z.B. das 5'-Methyl-, 3',5'-Di-t-butyl-, 5'-t-Butyl-, 5'-(1,1,3,3-Tetramethylbutyl)-, 5-Chlor-3',5'-di-t-butyl-, 5-Chlor-3'-t-butyl-5'-methyl-, 3'-sec.Butyl-5'-t-butyl, 4'-Octoxy-, 3',5'-Di-t-amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-t-Butyl-phenylsalicylat, Phenylsalicylat, Octylphenylsalicylat, Dibenzoylresorcin, Bis-(4-t-butylbenzoyl)-resorcin, Benzoylresorcin, 3,5-Di-t-butyl-4-hydroxybenzoesäure-2,4-di-t-butylphenylester, 3,5-Di-t-butyl-4-hydroxybenzoesäurehexadecylester.

2.4. Acrylate, wie z.B. α-Cyan-β,β-diphenylacrylsäure-äthylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triäthanolamin oder N-Cyclohexyl-diäthanolamin, Nickeldibutyldithiocarbamat, Nickelsalze von 4-Hydroxy-3,5-di-t-butylbenzylphosphonsäure-monoalkylestern wie vom Methyl- oder Aethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethylpiperidyl)-sebacat Bis-(1,2,2,6,6-pentamethylpiperidyl)-sebacat n-Butyl-3,5-di-t-butyl-4-hydroxybenzyl-malonsäure-bis(1,2,2,6,6-pentamethylpiperidyl)-ester, Kondensationsprodukt aus 1-Hydroxyäthyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-t-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1'-(1,2-Aethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-t-butyl-oxanilid, 2,2'-Di-dodecyloxy-5,5'-di-t-butyl-oxanilid, 2-Aethoxy-2'-äthyl-oxanilid, N,N'-Bis-(3-dimethylaminopropyl)-oxalamid, 2-Aethoxy-5-t-butyl-2'-äthyloxanilid und dessen Gemisch mit 2-Aethoxy-2'-äthyl-5,4'-di-t-butyl-oxanilid, Gemische von o-und p-Methoxy- sowie von o- und p-Aethoxy-di-substituierte Oxaniliden.

3. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-t-butylphenyl)-phosphit, Diisodecylpentaerythrit-diphosphit, Di-(2,4-di-t-butylphenyl)-pentaerythritdiphosphit, Tristearyl-sorbit-triphosphit, Tetrakis-(2,4-di-t-butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

4. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

5. Polyamidstabilisatoren, wie z.B. Kupfersalze in Kombination mit Jodiden und/oder Phosphorverbindungen und Salze des zweiwertigen Mangans.

6. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

7. Nukleierungsmittel, wie z.B. 4-tert.Butylbenzoesäure, Adipinsäure, Diphenylessigsäure.

8. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

9. Sonstige Zusätze, wie z.B. Weichmacher, Gleitmittel, Emulgatoren, Pigmente, optische Aufheller, Flammschutzmittel, Antistatika, Treibmittel, Wachse, Oele, organische Lösungsmittel.

Die nachfolgenden Beispiele erläutern die Erfindung weiter. In den Beispielen, der übrigen Beschreibung und den Patentansprüchen genannte Prozente und Teile sind Gewichtsprozente und Gewichtsteile.

HERSTELLUNGSBEISPIEL

Beispiel 1: Herstellung von 2,4-Bis(n-octylthiomethyl)-6-methylphenol (Verbindung Nr. 1)

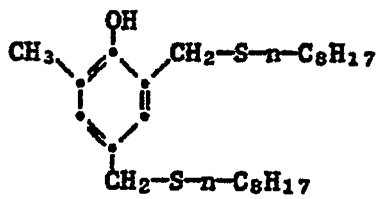

160,74 g(0,72 Mol) 2,4-Bis(dimethylaminomethyl)-6-methylphenol und 210,65 g (1,44 Mol) n-Octanthiol werden in einer Apparatur mit Rührer und Intensivkühler 36 Stunden lang auf 150°C erhitzt, wobei bei 53,2 bar laufend Dimethylamin abgezogen wird. Man erhält 291,6 g (95 %) eines gelben Oels. Reines 2,4-Bis(n-octylthiomethyl)-6-methyl-phenol wird durch Säulenchromatographie des Rohproduktes an Kieselgel als farbloses Oel erhalten.

**Analysewerte:**

| | | | |
|---|---|---|---|
| Berechnet | 70,69 % C | Gefunden | 70,85 % C |
| | 10,44 % H | | 10,42 % H |
| | 15,09 % S | | 15,11 % S |

$^1$H-NMR ($CDCl_3$; 100 MHz; Angaben in δ [ppm] bezogen auf TMS = O, s = Singlett): Charakteristische Signale für $Ar-CH_2S$ bei 3,77 (s) und 3,6 (s).

Analogerweise werden die nachfolgenden Verbindungen der Formel I hergestellt:

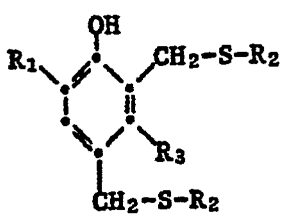

(I)

| Verbindung Nr. | $R_1$ | $R_2$ | $R_3$ | Charakteristika |
|---|---|---|---|---|
| Verbindung 2 | $CH_3$ | $n-C_{12}H_{25}$ | H | Smp.: 28°C |
| Verbindung 3 | $CH_3$ | $n-C_{12}H_{25}$ | $CH_3$ | Smp.: 43°C |
| Verbindung 4 | $C(CH_3)_3$ | $n-C_{12}H_{25}$ | $CH_3$ | Smp.: 40°C |
| Verbindung 5 | $CH_3$ | $n-C_8H_{17}$ | $CH_3$ | flüssig |
| Verbindung 6 | $CH_3$ | 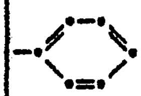 | H | Smp.: 99-100°C |
| Verbindung 7 | $CH_3$ | $-CH_2-$ 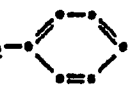 | H | flüssig |
| Verbindung 8 | $CH_3$ | $-CH_2-COO-CH_2CH(CH_2CH_3)-(CH_2)_3-CH_3$ | H | flüssig |
| Verbindung 9 | $CH_3$ | $-CH_2CH_2-OH$ | H | flüssig |
| Verbindung 10 | $CH_3$ | $-C(CH_3)_2-CH_2-C(CH_3)_2-CH_2-C(CH_3)_3$ | H | flüssig |
| Verbindung 11 | $CH_3$ | $-C(CH_3)_2-CH_2-C(CH_3)_3$ | H | flüssig |

## ANWENDUNGSBEISPIELE

Die Verbindungen 1-4 aus Beispiel 1 werden auf ihre Stabilisatorwirkung hin geprüft.

Beispiel 2: Stabilisierung von Polybutadien-Kautschuk (Ofenalterung)

100 g Polybutadien, welches mit 0,4 % 2,6-Di-t-butyl-p-cresol vorstabilisiert ist, werden auf einem Mischwalzwerk bei 50°C während 6 Minuten homogen mit 0,25 % der zu prüfenden Verbindung gemischt. Aus den Walzfallen werden bei 80°C Platten von 10 mm Dicke gepresst. Eine weitere Platte wird ohne Stabilisator auf dieselbe Weise hergestellt.

Die Prüfung der erdindungsgemässen Zusammensetzungen wird durch Hitzealterung in einem Umluftofen bei 80°C vorgenommen. Als Kriterium dient der während der Ofenalterung auftretende unerwünschte Gelgehalt. Dieser wird folgendermassen bestimmt:
1 Gramm Polybutadien wird über Nacht bei Raumtemperatur in 100 ml Toluol gelöst. Die Lösung wird durch eine Glasfilternutsche (Porengrösse 00) filtriert und die filtrierte Lösung zur Trockne eingedampft.

Der Gelgehalt ergibt sich aus

$$\text{Gel} = \frac{E - A}{E} \times 100\ (\%)$$

E = Einwaage (1 Gramm)
A = Gewicht des Eindampfrückstandes
Der Gelgehalt nimmt nach einer Induktionsperiode rasch zu. Als willkürliche Definition der Induktionsperiode dient die Zeit, nach welcher ein Gelgehalt von 2 % erreicht wird. Diese Induktionszeit wird durch periodische Gelgehaltsbestimmungen ermittelt.

Die Resultate sind in Tabelle 1 zusammengefasst.

Tabelle 1

| Stabilisator | Induktionszeit bis Gelgehalt = 2 % (in Tagen) |
|---|---|
| Keiner | 21 |
| Verbindung 2 | 77 |
| Verbindung 3 | 84 |
| Verbindung 4 | 56 |

Beispiel 3: Stabilisierung von Polybutadien-Kautschuk (Brabender)

100 Teile Polybutadien, welches mit 0,4 % 2,6-Di-t-butyl-p-cresol vorstabilisiert ist, wird zusätzlich mit 0,25 % des zu prüfenden Stabilisators in einem Brabender-Plastographen bei 160°C und 60 Umdrehungen pro Minute während 30 Minuten geknetet. Aus dem Verlauf der Drehmomentskurve wird die Induktionszeit ermittelt, d.h. die Knetzeit in Minuten bis zum Anstieg des Drehmoments um 100 mp nach dem Drehmomentminimum.

Die Ergebnisse sind in Tabelle 2 zusammengefasst.

Tabelle 2

| Stabilisator | Induktionszeit (in Minuten) |
|---|---|
| keiner | 8 |
| Verbindung 1 | > 30 |
| Verbindung 2 | 23 |
| Verbindung 3 | 23,5 |
| Verbindung 4 | 26 |

Beispiel 4: TFOUT-Test (Thin-Film Oxygen Uptake Test)

Dieser Test ist eine modifizierte Version des "Rotary Bomb Oxidationstests für Mineralöle" (ASTM D 2272). Er wird genau beschrieben in "C.S.Ku und S.M.Hsu, A Thin-Film Oxygen Uptake Test for the Evaluation of Automotive Crankcase Lubricants, Lubrication Engineering, Vol. 40 (2), 75-83 (1984". Als Testöl dient ein Motorenöl auf Mineralölbasis, welches die Hälfte der üblichen Menge an Zinkdithiophosphat (0,75 %; Zinkgehalt 0,06 %, bezogen auf das Motorenöl) enthält; diese Aenderung wird vorgenommen, damit ein potentieller Effekt des zu prüfenden Stabilisators gezeigt werden kann.

Die Verbindung 1 aus Beispiel 1 wird im beschriebenen Motorenöl in Gegenwart von 2 % Wasser, einer flüssigen oxidierten, nitrierten Fraktion eines Motorenbenzins als Katalysator (4 % Einsatzkonzentration), eines flüssigen Metallnaphthenates als weiteren Katalysator (4 % Einsatzkonzentration; Wasser und die beiden flüssigen Katalysatorsubstanzen sind unter der No. Standard Reference Material 1817 vom National Bureau of Standards (NBS) mit Analysen-Zertifikat geliefert worden) getestet. Der Versuch ist bei einem deutlichen Knick des Druck/Zeit-Diagramms beendet. Die in der untenstehenden Tabelle angegebenen Resultate bedeuten die Zeit (in Minuten) bis zum Knick des Druck/Zeit-Diagramms.

Lange Zeiten entsprechen guter Stabilisatorwirksamkeit. Konzentration des Stabilisators: 0,5 %, bezogen auf das Oel.

Tabelle 3

| Stabilisator | Minuten bis deutlicher Druckabfall |
|---|---|
| keiner | 85 |
| Verbindung 1 | 134 |

Beispiel 5: Stabilisierung eines hydrierten $C_9$-Kohlenwasserstoffharzes (Ofenalterung)

50 g Harz, welches mit 0,15 % 2,6-Di-t-butyl-p-cresol vorstabilisiert ist, werden in einem Glaskolben bei 170°C geschmolzen und 0,1 % der Verbindung 1 während 15 Minuten unter Rühren eingemischt. Die heisse Schmelze wird dann in Aluminiumschalen bzw. Petrischalen abgefüllt. Die Schichtdicke der erstarrten Harzmasse beträgt in den Aluminiumschalen ca. 15 mm und in den Petrischalen 1 mm. Die Prüfung der erfindungsmässigen Zusammensetzung wird durch Hitzealterung in einem Umluftofen bei 170°C vorgenommen. Als Kriterium dient die während der Ofenalterung auftretende unerwünschte Verfärbung. Diese wird folgendermassen bestimmt:

a) bei den 1 mm-Proben: Yellowness Index nach ASTM D 1925-70 und
b) bei den 15 mm-Proben: Gardner-Farbe nach DIN 6161.

Höhere Zahlen bedeuten jeweils stärkere Verfärbung.
Die Resultate sind in Tabelle 4 zusammengefasst.

Tabelle 4

| Stabilisator Nr. | Yellowness Index nach Stunden bei 170°C | | | Gardner-Farbe nach Stunden bei 170°C | | |
|---|---|---|---|---|---|---|
| | 0 | 12 | 30 | 0 | 12 | 30 |
| keiner | 3 | 86 | 126 | 1 | 5 | 9 |
| Verbindung 1 | 3 | 6 | 12 | 1 | 1-2 | 3-4 |

**Patentansprüche für die Vertragsstaaten: BE, CH, DE, FR, GB, IT, LI, NL**

1. Zusammensetzungen enthaltend ein Elastomer oder ein klebrigmachendes Harz und mindestens eine Verbindung der Formel I

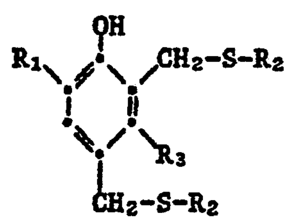

(I),

worin
$R_1$ $C_1$-$C_8$-Alkyl oder $C_5$-$C_{12}$-Cycloalkyl bedeutet,
die Reste $R_2$ unabhängig voneinander für $C_1$-$C_{18}$-Alkylgruppen stehen, welche unsubstituiert sind, oder durch 1 oder 2 Hydroxylgruppen oder durch -OCO-$R_4$, -CO-$OR_5$ oder -CO-$NR_5R_6$ substituiert sind, oder Phenyl oder Benzyl bedeuten,
$R_3$ Wasserstoff oder Methyl bedeutet, unter der Bedingung, dass, wenn $R_1$ von Methyl verschieden ist, $R_3$ für Methyl steht,

$R_4$ $C_1$-$C_{17}$-Alkyl, $C_2$-$C_8$-Alkenyl oder ein Rest der Formel Ia ist

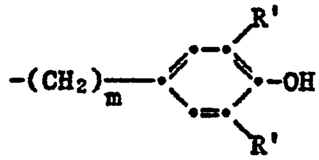

(Ia),

m für 0, 1 oder 2 steht, und die Reste
R' unabhängig voneinander die für $R_1$ genannte Bedeutung haben,
$R_5$ ein gegebenenfalls durch -O-, -S- oder -NR'- unterbrochenes $C_1$-$C_{20}$-Alkyl ist, und
$R_6$ für Wasserstoff steht oder eine für $R_5$ genannte Bedeutung besitzt.

2. Zusammensetzungen gemäss Anspruch 1, enthaltend ein Elastomer und mindestens eine Verbindung der Formel I gemäss Anspruch 1.

3. Zusammensetzungen gemäss Anspruch 2, worin die Reste $R_2$ unabhängig voneinander für $C_2$-$C_{18}$-Alkylgruppen stehen, welche unsubstituiert sind, oder durch 1 oder 2 Hydroxylgruppen oder durch -OCO-$R_4$, -CO-$OR_5$ oder -CO-$NR_5R_6$ substituiert sind, oder Benzyl oder Phenyl bedeuten, wobei $R_4$, $R_5$ und $R_6$ die in Anspruch 1 genannte Bedeutung haben.

4. Zusammensetzungen gemäss Anspruch 1, worin $R_1$ $C_1$-$C_4$-Alkyl ist.

5. Zusammensetzungen gemäss Anspruch 2, worin $R_1$ Methyl oder t-Butyl ist.

6. Zusammensetzungen gemäss Anspruch 1, worin $R_2$ $C_4$-$C_{18}$-Alkyl, $-CH_2CH_2-OH$, $-CH_2CH_2-OCO-R_4$, $-CH_2-COO-R_5$ oder $-CH_2CH_2-COO-R_5$ Benzyl oder Phenyl bedeutet, wobei $R_4$ und $R_5$ die in Anspruch 1 genannte Bedeutung haben.

7. Zusammensetzungen gemäss Anspruch 6, worin $R_2$ $C_4$-$C_{18}$-Alkyl bedeutet.

8. Zusammensetzungen gemäss Anspruch 1, worin das Elastomer ein Polydien, ein halogenhaltiges Polymer oder ein Polyurethan ist.

9. Zusammensetzungen gemäss Anspruch 1, enthaltend 0,05-5,0 Gew.-% des Stabilisators der Formel I, bezogen auf das Elastomer bzw. auf das klebrigmachende Harz.

10. Verbindungen der Formel II

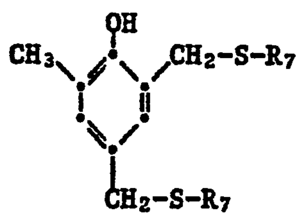

(II),

worin die Reste $R_7$ unabhängig voneinander für $C_8$-$C_{18}$-Alkylgruppen stehen, welche unsubstituiert sind oder durch 1 oder 2 Hydroxylgruppen oder durch -OCO-$R_4$, -CO-$OR_5$ oder -CO-$NR_5R_6$ substituiert sind, oder Phenyl oder Benzyl bedeuten, wobei $R_4$, $R_5$ und $R_6$ die in Anspruch 1 genannte Bedeutung haben.

11. Verbindungen der Formel II gemäss Anspruch 10, worin die Reste $R_7$ unabhängig voneinander für unsubstituiertes $C_8$-$C_{18}$-Alkyl stehen.

12. Verwendung von Verbindungen der Formel II als Stabilisatoren für Schmierstoffe gegen deren Schädigung durch Einwirkung von Sauerstoff, Wärme, Licht und energiereiche Strahlung.

13. Zusammensetzungen enthaltend einen Schmierstoff und mindestens eine Verbindung der Formel II gemäss Anspruch 10.

**Patentansprüche für den Vertragsstaat: ES**

1. Zusammensetzungen enthaltend ein Elastomer oder ein klebrigmachendes Harz und mindestens eine Verbindung der Formel I

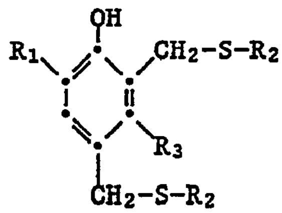

(I),

worin
$R_1$ $C_1$–$C_8$-Alkyl oder $C_5$–$C_{12}$-Cycloalkyl bedeutet,
die Reste $R_2$ unabhängig voneinander für $C_1$–$C_{18}$-Alkylgruppen stehen, welche unsubstituiert sind, oder durch 1 oder 2 Hydroxylgruppen oder durch $-OCO-R_4$, $-CO-OR_5$ oder $-CO-NR_5R_6$ substituiert sind, oder Phenyl oder Benzyl bedeuten,
$R_3$ Wasserstoff oder Methyl bedeutet, unter der Bedingung, daß, wenn $R_1$ von Methyl verschieden ist, $R_3$ für Methyl steht,
$R_4$ $C_1$–$C_{17}$-Alkyl, $C_2$–$C_8$-Alkenyl oder ein Rest der Formel Ia ist

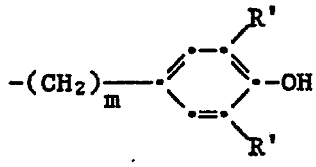

(Ia),

m für 0,1 oder 2 steht, und die Reste
R' unabhängig voneinander die für $R_1$ genannte Bedeutung haben,
$R_5$ ein gegebenenfalls durch –O–, –S– oder –NR'– unterbrochenes $C_1$–$C_{20}$-Alkyl ist, und
$R_6$ für Wasserstoff steht oder eine für $R_5$ genannte Bedeutung besitzt.

2. Zusammensetzungen gemäß Anspruch 1, enthaltend ein Elastomer und mindestens eine Verbindung der Formel I gemäß Anspruch 1.

3. Zusammensetzungen gemäß Anspruch 2, worin die Reste $R_2$ unabhängig voneinander für $C_2$–$C_{18}$-Alkylgruppen stehen, welche unsubstituiert sind, oder durch 1 oder 2 Hydroxylgruppen oder durch $-OCO-R_4$, $-CO-OR_5$ oder $-CO-NR_5R_6$ substituiert sind, oder Benzyl oder Phenyl bedeuten, wobei $R_4$, $R_5$ und $R_6$ die in Anspruch 1 genannte Bedeutung haben.

4. Zusammensetzungen gemäß Anspruch 1, worin $R_1$ $C_1$–$C_4$-Alkyl ist.

5. Zusammensetzungen gemäß Anspruch 2, worin $R_1$ Methyl oder t-Butyl ist.

6. Zusammensetzungen gemäß Anspruch 1, worin $R_2$ $C_4$–$C_{18}$-Alkyl, $-CH_2CH_2-OH$, $-CH_2CH_2-OCO-R_4$, $-CH_2-COO-R_5$ oder $-CH_2CH_2-COO-R_5$, Benzyl oder Phenyl bedeutet, wobei $R_4$ und $R_5$ die in Anspruch 1 genannte Bedeutung haben.

7. Zusammensetzungen gemäß Anspruch 6, worin $R_2$ $C_4$–$C_{18}$-Alkyl bedeutet.

8. Zusammensetzungen gemäß Anspruch 1, worin das Elastomer ein Polydien, ein halogenhaltiges Polymer oder ein Polyurethan ist.

9. Zusammensetzungen gemäß Anspruch 1, enthaltend 0,05–5,0 Gew.-% des Stabilisators der Formel I, bezogen auf das Elastomer bzw. auf das klebrigmachende Harz.

10. Verfahren zur Herstellung von Verbindungen der Formel II

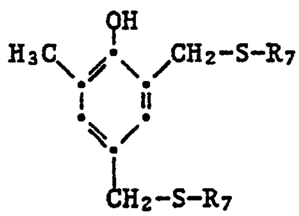

(II),

worin die Reste $R_7$ unabhängig voneinander für $C_8$–$C_{18}$-Alkylgruppen stehen, welche unsubstituiert sind oder durch 1 oder 2 Hydroxylgruppen oder durch $-OCO-R_4$, $-CO-OR_5$ oder $-CO-NR_5R_6$ substituiert sind, oder Phenyl oder Benzyl bedeuten,
$R_4$ $C_1$–$C_{17}$-Alkyl, $C_2$–$C_8$-Alkenyl oder ein Rest der Formel Ia ist

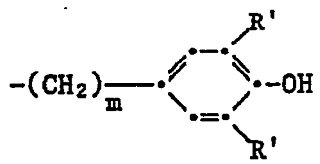 (Ia),

m für 0, 1 oder 2 steht, und die Reste R' $C_1$–$C_8$-Alkyl oder $C_5$–$C_{12}$-Cycloalkyl bedeuten, $R_5$ ein gegebenenfalls durch –O–, –S– oder –NR'– unterbrochenes $C_1$–$C_{20}$-Alkyl ist, und $R_6$ für Wasserstoff steht oder eine für $R_5$ genannte Bedeutung besitzt, dadurch gekennzeichnet, daß man 2,4-Bis(dimethylaminomethyl)-6-methylphenol mit einem Merkaptan $R_7$–SH umsetzt.

11. Verfahren gemäß Anspruch 10, worin die Reste $R_7$ unabhängig voneinander für unsubstituiertes $C_8$–$C_{18}$-Alkyl stehen.

12. Zusammensetzungen enthaltend einen Schmierstoff und mindestens eine Verbindung der Formel II gemäß Anspruch 10.

**Claims for the Contracting States: BE, CH, DE, FR, GB, IT, LI, NL**

1. A composition containing an elastomer or a tackifying resin and at least one compound of formula I

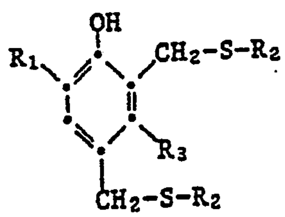 (I)

wherein
$R_1$ is $C_1$–$C_8$alkyl or $C_5$–$C_{12}$cycloalkyl, the radicals $R_2$, each independently of the other, are $C_1$–$C_{18}$alkyl groups which are unsubstituted or substituted by 1 or 2 hydroxyl groups or by –OCO–$R_4$, –CO–$OR_5$ or –CO–$NR_5R_6$, or they are phenyl or benzyl, $R_3$ is hydrogen or methyl, with the proviso that, if $R_1$ is other than methyl, then $R_3$ is methyl, $R_4$ is $C_1$–$C_{17}$alkyl, $C_2$–$C_8$alkenyl or a radical of formula Ia

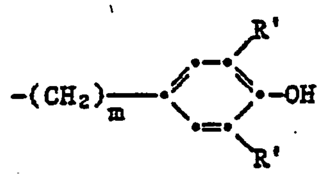 (Ia)

m is 0, 1 or 2, the radicals R', each independently of the other, have the meanings indicated for $R_1$, $R_5$ is $C_1$–$C_{20}$alkyl which may be interrupted by –O–, –S– or –NR'–, and $R_6$ is hydrogen or has one of the meanings indicated for $R_5$.

2. A composition according to claim 1, containing an elastomer and at least one compound of formula I according to claim 1.

3. A composition according to claim 2, wherein the radicals $R_2$, each independently of the other, are $C_2$–$C_{18}$alkyl groups which are unsubstituted or substituted by 1 or 2 hydroxyl groups or by –OCO–$R_4$, –CO–$OR_5$ or –CO–$NR_5R_6$, or they are benzyl or phenyl, with $R_4$, $R_5$ and $R_6$ being as defined in claim 1.

4. A composition according to claim 1, wherein $R_1$ is $C_1$–$C_4$-alkyl.

5. A composition according to claim 2, wherein $R_1$ is methyl or tert-butyl.

6. A composition according to claim 1, wherein $R_2$ is $C_4$–$C_{18}$alkyl, –$CH_2CH_2$–OH, –$CH_2CH_2$–OCO–$R_4$, –$CH_2$–COO–$R_5$ or –$CH_2CH_2$–COO–$R_5$, or benzyl or phenyl, with $R_4$ and $R_5$ being as defined in claim 1.

7. A composition according to claim 6, wherein $R_2$ is $C_4$–$C_{18}$alkyl.

8. A composition according to claim 1, wherein the elastomer is a polydiene, a halogen-containing polymer or a polyurethane.

9. A composition according to claim 1, containing 0.05 to 5.0% by weight of the stabiliser of formula I, based on the elastomer or the tackifying resin.

10. A compound of formula II

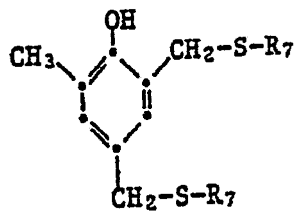

(II)

wherein the radicals $R_7$, each independently of the other, are $C_8$–$C_{18}$alkyl groups which are unsubstituted or substituted by 1 or 2 hydroxyl groups or by $-OCO-R_4$, $-CO-OR_5$ or $-CO-NR_5R_6$, or they are phenyl or benzyl, with $R_4$, $R_5$ and $R_6$ being as defined in claim 1.

11. A compound of formula II according to claim 10, wherein the radicals $R_7$, each independently of the other, are unsubstituted $C_8$–$C_{18}$alkyl.

12. Use of a compound of formula II for stabilising lubricants against the action of oxygen, heat, light and high-energy radiation.

13. A composition containing a lubricant and at least one compound of formula II according to claim 10.

**Claims for the Contracting State: ES**

1. A composition containing an elastomer or a tackifying resin and at least one compound of formula I

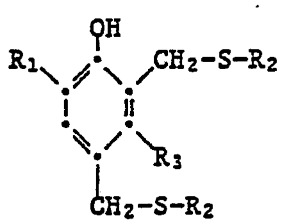

(I)

wherein
$R_1$ is $C_1$–$C_8$alkyl or $C_5$–$C_{12}$cycloalkyl, the radicals $R_2$, each independently of the other, are $C_1$–$C_{18}$alkyl groups which are unsubstituted or substituted by 1 or 2 hydroxyl groups or by $-OCO-R_4$, $-CO-OR_5$ or $-CO-NR_5R_6$, or they are phenyl or benzyl, $R_3$ is hydrogen or methyl, with the proviso that, if $R_1$ is other than methyl, then $R_3$ is methyl, $R_4$ is $C_1$–$C_{17}$alkyl, $C_2$–$C_8$alkenyl or a radical of formula Ia

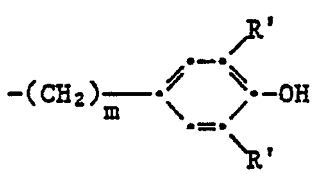

(Ia)

m is 0, 1 or 2, the radicals R', each independently of the other, have the meanings indicated for $R_1$, $R_5$ is $C_1$–$C_{20}$alkyl which may be interrupted by –O–, –S– or –NR'–, and $R_6$ is hydrogen or has one of the meanings indicated for $R_5$.

2. A composition according to claim 1, containing an elastomer and at least one compound of formula I according to claim 1.

3. A composition according to claim 2, wherein the radicals $R_2$, each independently of the other, are $C_2$–$C_{18}$alkyl groups which are unsubstituted or substituted by 1 or 2 hydroxyl groups or by $-OCO-R_4$, $-CO-OR_5$ or $-CO-NR_5R_6$, or they are benzyl or phenyl, with $R_4$, $R_5$ and $R_6$ being as defined in claim 1.

4. A composition according to claim 1, wherein $R_1$ is $C_1$–$C_4$alkyl.

5. A composition according to claim 2, wherein $R_1$ is methyl or tert-butyl.

6. A composition according to claim 1, wherein $R_2$ is $C_4$–$C_{18}$alkyl, $-CH_2CH_2-OH$, $-CH_2CH_2-OCO-R_4$, $-CH_2-COO-R_5$ or $-CH_2CH_2-COO-R_5$, or benzyl or phenyl, with $R_4$ and $R_5$ being as defined in claim 1.

7. A composition according to claim 6, wherein $R_2$ is $C_4$–$C_{18}$alkyl.

8. A composition according to claim 1, wherein the elastomer is a polydiene, a halogen-containing polymer or a polyurethane.

9. A composition according to claim 1, containing 0.05 to 5.0% by weight of the stabiliser of formula I, based on the elastomer or the tackifying resin.

10. A process for the preparation of a compound of formula II

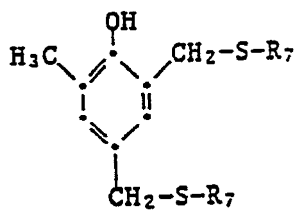

(II)

wherein the radicals $R_7$, each independently of the other, are $C_8$–$C_{18}$alkyl groups which are unsubstituted or substituted by 1 or 2 hydroxyl groups or by $-OCO-R_4$, $-CO-OR_5$ or $-CO-NR_5R_6$, or they are phenyl or benzyl, $R_4$ is $C_1$–$C_{17}$alkyl, $C_2$–$C_8$alkenyl or a radical of formula Ia

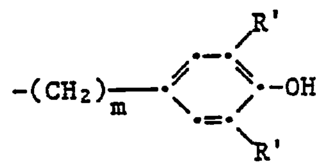

(Ia)

m is 0, 1 or 2, and the radicals R' are $C_1$–$C_8$alkyl or $C_5$–$C_{12}$cycloalkyl, $R_5$ is $C_1$–$C_{20}$alkyl which may be interrupted by –O–, –S– or –NR'–, and $R_6$ is hydrogen or has one of the meanings indicated for $R_5$, which comprises reacting 2,4-bis(dimethylaminomethyl)-6-methylphenol with a mercaptan $R_7$–SH.

11. A process according to claim 10, wherein the radicals $R_7$, each independently of the other, are unsubstituted $C_8$–$C_{18}$alkyl.

12. A composition containing a lubricant and at least one compound of formula II according to claim 10.

**Revendications pour les Etats contractants: BE, CH, DE, FR, GB, IT, LI, NL**

1. Compositions qui contiennent un élastomère ou une résine colle et au moins un composé répondant à la formule I:

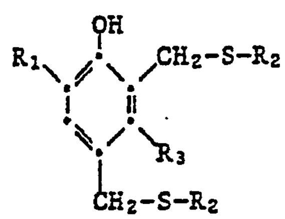

(I),

dans laquelle:

$R_1$ représente un alkyle en $C_1$–$C_8$ ou un cycloalkyle en $C_5$–$C_{12}$,

les $R_2$ représentent chacun, indépendamment l'un de l'autre, un alkyle en $C_1$–$C_{18}$, non substitué ou porteur d'un ou deux radicaux hydroxy ou porteur d'un radical $-OCO-R_4$, $-CO-OR_5$ ou $-CO-NR_5R_6$, un phényle ou un benzyle,

$R_3$ représente l'hydrogène ou un méthyle, avec la condition que, lorsque $R_1$ n'est pas un méthyle, $R_3$ soit un méthyle,

$R_4$ représente un alkyle en $C_1$–$C_{17}$, un alcényle en $C_2$–$C_8$ ou un radical répondant à la formule Ia:

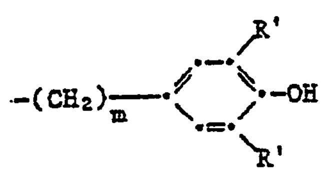

(Ia),

dans laquelle m est égal à 0, à 1 ou à 2 et les symboles R' ont chacun, indépendamment l'un de l'autre, la signification qui a été donnée pour $R_1$,

$R_5$ représente un radical alkyle en $C_1$–$C_{20}$ éventuellement interrompu par –O–, –S– ou –NR'–,

$R_6$ représente l'hydrogène ou a la signification qui a été donnée pour $R_5$.

2. Compositions selon la revendication 1 qui contiennent un élastomère et au moins un composé de formule I selon la revendication 1.

3. Compositions selon la revendication 2 dans lesquelles les $R_2$ représentent chacun, indépendamment l'un de l'autre, un alkyle en $C_2$–$C_{18}$ non substitué ou porteur d'un ou de deux radicaux hydroxy ou d'un radical $-OCO-R_4$, $-CO-OR_5$ ou $-CO-NR_5R_6$, un benzyle ou un phényle, les symboles $R_4$, $R_5$ et $R_6$ ayant les significations qui leur ont été données à la revendication 1.

4. Compositions selon la revendication 1 dans lesquelles $R_1$ représente un alkyle en $C_1$–$C_4$.

5. Compositions selon la revendication 2 dans lesquelles $R_1$ représente un radical méthyle ou tert-butyle.

6. Compositions selon la revendication 1 dans lesquelles $R_2$ représente un alkyle en $C_4$–$C_{18}$, $-CH_2CH_2-OH$, $-CH_2CH_2-OCO-R_4$, $-CH_2-COO-R_5$ ou $-CH_2CH_2-COO-R_5$, un benzyle ou un phényle, les symboles $R_4$ et $R_5$ ayant les significations qui leur ont été données à la revendication 1.

7. Compositions selon la revendication 6 dans lesquelles $R_2$ représente un alkyle en $C_4$–$C_{18}$.

8. Compositions selon la revendication 1 dans lesquelles l'élastomère est un polydiène, un polymère halogéné ou un polyuréthanne.

9. Compositions selon la revendication 1 qui contiennent de 0,05 à 5,0% en poids du stabilisant de formule I, par rapport à l'élastomère ou à la résine colle.

10. Composés répondant à la formule II

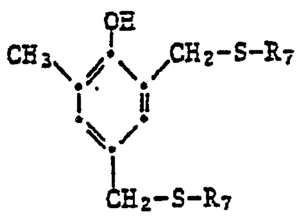

(II),

dans laquelle les $R_7$ représentent chacun, indépendamment l'un de l'autre, un alkyle en $C_8$–$C_{18}$ non substitué ou porteur d'un ou de deux radicaux hydroxy ou d'un radical $-OCO-R_4$, $-CO-OR_5$ ou $-CO-NR_5R_6$, un phényle ou un benzyle, les symboles $R_4$, $R_5$ et $R_6$ ayant les significations qui leur ont été données à la revendication 1.

11. Composés de formule II selon la revendication 10 dans lesquels les $R_7$ représentent chacun, indépendamment l'un de l'autre, un radical alkyle en $C_8$–$C_{18}$ qui ne porte pas de substituant.

12. Application de composés de formule II comme stabilisants pour des lubrifiants à protéger contre la dégradation due à l'action de l'oxygène, de la chaleur, de la lumière et de rayonnements de haute énergie.

13. Compositions qui contiennent un lubrifiant et au moins un composé de formule II selon la revendication 10.

**Revendications pour l'Etat Contractant: ES**

1. Compositions qui contiennent un élastomère ou une résine colle et au moins un composé répondant à la formule I:

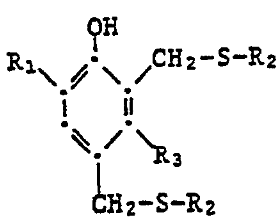

(I),

dans laquelle:

$R_1$ représente un alkyle en $C_1$–$C_8$ ou un cycloalkyle en $C_5$–$C_{12}$,

les $R_2$ représentent chacun, indépendamment l'un de l'autre, un alkyle en $C_1$–$C_{18}$, non substitué ou porteur d'un ou deux radicaux hydroxy ou porteur d'un radical $-OCO-R_4$, $-CO-OR_5$ ou $-CO-NR_5R_6$, un phényle ou un benzyle,

$R_3$ représente l'hydrogène ou un méthyle, avec la condition que, lorsque $R_1$ n'est pas un méthyle, $R_3$ soit un méthyle,

$R_4$ représente un alkyle en $C_1$–$C_{17}$, un alcényle en $C_2$–$C_8$ ou un radical répondant à la formule Ia:

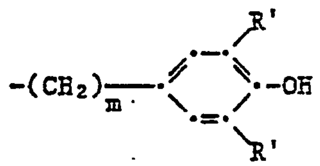

(Ia),

dans laquelle m est égal à 0, à 1 ou à 2 et les symboles R' ont chacun, indépendamment l'un de l'autre, la signification qui a été donnée pour $R_1$,
$R_5$ représente un radical alkyle en $C_1$–$C_{20}$ éventuellement interrompu par –O–, –S– ou –NR'–, et
$R_6$ représente l'hydrogène ou a la signification qui a été donnée pour $R_5$.

2. Compositions selon la revendication 1 qui contiennent un élastomère et au moins un composé de formule I selon la revendication 1.

3. Compositions selon la revendication 2 dans lesquelles les $R_2$ représentent chacun, indépendamment l'un de l'autre, un alkyle en $C_2$–$C_{18}$ non substitué ou porteur d'un ou de deux radicaux hydroxy ou d'un radical $-OCO-R_4$, $-CO-OR_5$ ou $-CO-NR_5R_6$, un benzyle ou un phényle, les symboles $R_4$, $R_5$ et $R_6$ ayant les significations qui leur ont été données à la revendication 1.

4. Compositions selon la revendication 1 dans lesquelles $R_1$ représente un alkyle en $C_1$–$C_4$.

5. Compositions selon la revendication 2 dans lesquelles $R_1$ représente un radical méthyle ou tert-butyle.

6. Compositions selon la revendication 1 dans lesquelles $R_2$ représente un alkyle en $C_4$–$C_{18}$, $-CH_2CH_2-OH$, $-CH_2CH_2-OCO-R_4$, $-CH_2-COO-R_5$ ou $-CH_2CH_2-COO-R_5$, un benzyle ou un phényle, les symboles $R_4$ et $R_5$ ayant les significations qui leur ont été données à la revendication 1.

7. Compositions selon la revendication 6 dans lesquelles $R_2$ représente un alkyle en $C_4$–$C_{18}$.

8. Compositions selon la revendication 1 dans lesquelles l'élastomère est un polydiène, un polymère halogéné ou un polyuréthanne.

9. Compositions selon la revendication 1 qui contiennent de 0,05 à 5,0% en poids du stabilisant de formule I, par rapport à l'élastomère ou à la résine colle.

10. Procédé pour préparer des composés de formule II

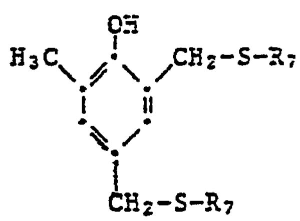

(II),

dans laquelle les $R_7$ représentent chacun, indépendamment l'un de l'autre, un alkyle en $C_8$–$C_{18}$ non substitué ou porteur d'un ou de deux radicaux hydroxy ou d'un radical $-OCO-R_4$, $-CO-OR_5$ ou $-CO-NR_5R_6$, un phényle ou un benzyle,
$R_4$ représente un alkyle en $C_1$–$C_{17}$, un alcényle en $C_2$–$C_8$ ou un radical répondant à la formule Ia

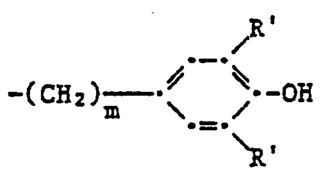

(Ia),

dans laquelle m est égal à 0, à 1 ou à 2 et les symboles R' représentent chacun un alkyle en $C_1$–$C_8$ ou un cycloalkyle en $C_5$–$C_{12}$,
$R_5$ représente un alkyle en $C_1$–$C_{20}$ éventuellement interrompu par –O–, –S– ou –NR'–, et
$R_6$ représente l'hydrogène ou a l'une des significations qui ont été données pour $R_5$,
procédé caractérisé en qu'on fait réagir le bis-(diméthylaminométhyl)-2,4 méthyl-6 phénol avec un mercaptan $R_7$–SH.

11. Procédé selon la revendication 10, selon lequel les symboles $R_7$ représentent chacun, indépendamment l'un de l'autre, un alkyle en $C_8$–$C_{18}$ non substitué.

12. Compositions contenant un lubrifiant et au moins un composé de formule II selon la revendication 10.